(19) **Europäisches Patentamt / European Patent Office / Office européen des brevets**

(11) **EP 3 012 616 A1**

(12) **EUROPEAN PATENT APPLICATION**

(43) Date of publication:
**27.04.2016 Bulletin 2016/17**

(51) Int Cl.:
**G01N 21/17** (2006.01)          **G01N 33/28** (2006.01)
**E21B 49/00** (2006.01)

(21) Application number: **14290322.8**

(22) Date of filing: **22.10.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Services Petroliers Schlumberger**
  **75007 Paris (FR)**
  Designated Contracting States:
  **FR**
- **SAS AEROVIA**
  **51687 Reims Cedex 2 (FR)**
  Designated Contracting States:
  **FR**
- **Schlumberger Holdings Limited**
  **Road Town, Tortola 1110 (VG)**
  Designated Contracting States:
  **GB NL**
- **Schlumberger Technology B.V.**
  **2514 JG The Hague (NL)**
  Designated Contracting States:
  **AL AT BE BG CH CY CZ DE DK EE ES FI GR HR HU IE IS IT LI LT LU LV MC MK MT NO PL PT RO RS SE SI SK SM TR**

(72) Inventors:
- **Breviere, Jerôme**
  **95150 Taverny (FR)**
- **Colin, Lionel**
  **59153 Grand Fort Philippe (FR)**

(74) Representative: **Leonori, Céline**
**Etudes et Productions Schlumberger**
**Intellectual Property Department**
**1, rue Henri Becquerel**
**B.P. 202**
**92142 Clamart Cedex (FR)**

(54) **A system and method for analyzing a gaseous sample extracted from a drilling fluid coming from a wellbore**

(57) The disclosure relates to a system and method for analyzing a gaseous sample extracted from a drilling fluid coming from a wellbore. The system comprises a surface assembly, situated outside of the wellbore and including :
- an extractor for extracting a gaseous sample from the drilling fluid,
- a quantification device connected to the extractor for quantifying at least a gaseous constituent contained the gaseous sample.

The quantification device comprises at least a photoacoustic cell in fluid communication with the extractor, at least a light source for emitting a light beam at a predetermined emission spectrum in the photoacoustic cell, at least an acoustic detector positioned in the photoacoustic cell and a processor for calculating a parameter relative to the quantity of the gaseous constituent on the basis of a signal obtained from the detector.

FIG. 2

**Description**

**BACKGROUND**

This disclosure relates to a system and method for analyzing a gaseous sample extracted from a drilling fluid coming from a wellbore.

Such a system or method may analyze the gases extracted from a petroleum fluid produced in an oil well or determine extracted gas composition from a drilling fluid directly at the surface. This analysis allows the geological sequence of the formations passed through during the drilling operation to be reconstructed and is used to determine the possible applications of the fluid deposits encountered.

Such an analysis which is carried out in a continuous manner comprises two main phases. The first phase comprises extracting the gases carried by the mud (hydrocarbons, water ...). The second phase comprises qualifying and quantifying the extracted gases.

Analysis devices that are currently used can be a gas-phase chromatograph alone, a gas-phase chromatograph combined with an isotope ratio mass spectrometer (IRMS) or an infrared spectrometer.

It is desirable that the analysis devices give accurate results, detecting gaseous constituents even if the quantity of this gaseous constituent in the sample is low. It is also desirable that the analysis is fast, the device being able to analyze a maximum numbers of gaseous samples in real-time.

**SUMMARY**

A summary of certain embodiments disclosed herein is set forth below. It should be understood that these aspects are presented merely to provide the reader with a brief summary of these certain embodiments and that these aspects are not intended to limit the scope of this disclosure. Indeed, this disclosure may encompass a variety of aspects that may not be set forth below.

Embodiments of this disclosure relate to various systems, methods, and devices for analyzing a gaseous sample extracted from a drilling fluid coming from a wellbore.

A system for analyzing a gaseous sample extracted from a drilling fluid coming from a wellbore may comprise a surface assembly, situated outside of the wellbore and including an extractor for extracting a gaseous sample from the drilling fluid, and a quantification device connected to the extractor for quantifying gaseous constituents contained in the sample. The quantification device comprises a photoacoustic cell in fluid communication with the extractor, a light source for emitting a light beam having a predetermined emission spectrum in the photoacoustic cell, an acoustic detector positioned in the photoacoustic cell and a processor for calculating a parameter relative to the quantity of the gaseous constituent, such as a concentration of the gaseous constituent, on the basis of a signal obtained from the detector. The quantification is made isotopically and/or compositionally.

The emission spectrum of the light beam may comprise one predetermined wavelength value. It may also comprise several wavelength values.

Photoacoustic detection relies on periodically submitting molecules to a light beam, so that molecules absorb photons of the light beam, producing periodical increases of temperatures and pressure that create an acoustic wave. This acoustic wave is detected and may convey information relative to the molecules. The detected signal is proportional to the amount of the molecules that have absorbed the photons in the cell. This technique may therefore give information relative to the quantity of the gaseous constituents contained in the gaseous sample.

The analyzing system of the disclosure provides results in a few seconds.

The light sources may to emit two light beams having a distinct emission spectrum. In other words, the emission spectrum of one of the light beams may comprise wavelength values that are not included in the emission spectrum of the other light beams.

In particular, the system may comprise a plurality of light sources emitting light beams having a distinct emission spectrum. Each of the light sources may emit a light beam. The light sources may be associated with the same cell or to different cells.

The system may also comprise a light source having an adjustable emission spectrum. In other words, such a light source may emit a first light beam having a first emission spectrum and afterwards may emit a second light beam having a second distinct emission spectrum. Such a light source may be referred to as a tunable light source. Such a light source may be a broadly tunable light source. It can be used alone or in combination with other light sources that have an adjustable emission spectrum or not. An analysis system comprising a tunable light source may be compact while providing accurate results.

The quantifying device may comprise several cells, in series and/or parallel. A first light beam may be emitted in a first cell and a second light beam may be emitted in a second cell simultaneously. A first light beam may also be emitted in a first cell while no light beam is emitted in the second cell in particular when cells are in parallel. The cells may be connected so that a same gaseous sample flows through several cells.

The system may comprise a control module of the light sources for controlling the light sources so that a light beam is emitted at a time in a photoacoustic cell. Several light beams may however be emitted at the same time in a photoacoustic cell. The emission spectrum of the resulting light beam may then be determined.

The emission spectrum of the light beams emitted by the light sources may be focused on one wavelength value. It provides accurate detection. The light beams may have an emission spectrum comprising more than one wavelength value.

The power of the light beams may be greater than 1 mW. A gaseous constituent may be detected even if it is contained in the gaseous sample in a low quantity.

The or at least one of the light sources may comprise a LASER assembly. It may comprise a QCL (Quantum Cascade Laser) assembly. It may also comprise other type of LASER assemblies, such as for example a Gas LASER, a Semi-Conductor LASER, A Dye LASER or a Solid State LASER. Other types of LASER may also be used.

The processor may calculate the parameter relative to the quantity of a gaseous constituent in view of a first signal obtained from the acoustic detector corresponding to the emission of a first light beam and from a second signal obtained from the acoustic detector corresponding to the emission of a second light beam. The processor may then calculate the isotopic ratio of this constituent from the comparison of the first and second signals.

The processor may calculate the concentration of a gaseous constituent in view of the following formula:

$$\forall\, w_j,\ \sum_{i=1}^{n} A_{ij} \times x_i = X_j$$

$w_j$ is a predetermined measured wavelength value, $X_j$ the total concentration of gas detected for wavelength value $w_j$, $x_i$ the concentration of a gaseous constituent i and $A_{ij}$ is the fraction of the signal due to the constituent i at wavelength $w_j$. $A_{ij}$ may be determined beforehand via a calibration.

The above-mentioned relation may be obtained directly from the measures when the light beam has an emission spectrum comprising one wavelength.

When a light beam has a emission spectrum having several wavelength values, the relation may be as follows:

$$\sum_{j=1}^{m} B_j \sum_{i=1}^{n} A_{ij} \times x_i = \sum_{j=1}^{m} X_j$$

$X_j$ the total amount (for instance, quantity or concentration) of gas of interest detected for wavelength value $w_j$ (light beam comprising wavelengths $w_1,..., w_m$), $x_i$ the amount of a gaseous constituent i, $A_{ij}$ is the fraction of the signal due to the constituent i at wavelength $w_j$ and $B_j$ is the fraction of photons of the emission spectrum at wavelength $w_j$. $A_{ij}$ may be determined beforehand via a calibration and $B_j$ is known from the emission spectrum of the light beam.

The photoacoustic cell may be designed as a Helmholtz resonator having a predetermined acoustic resonant frequency.

The quantification device may also comprise a modulator associated to each light source so that it modulates the light beam, in particular at the acoustic resonant frequency of the Helmholtz resonator. The modulator may modulate the light source mechanically or electrically. It can modulate the light beams emitted by different light sources with different devices, of the same or different type, or a same device.

One of the wavelength values of the light sources may be chosen in the following areas :

- Between 3.30 and 3.45 $\mu$m,
- Between 6.67 and 6.75 $\mu$m
- Between 1, 58 and 1, 67 $\mu$m
- Between 7.52 and 7.70 $\mu$m
- Between 12, 05 and 12, 34 $\mu$m.

The emission spectrum of the light beams may be chosen so that the detector is able to detect gaseous constituents comprising carbon atoms, including an alkane with less than 15 carbon atoms and in particular methane (C1), ethane (C2), propane (C3) such as n-propane or isopropane, butane (C4) such as isobutane (iC4) or other isomers of butane, pentane (C5) such as isopentane (iC5) or other isomers of pentane. Heavier alkanes may also be detected by the system, as other constituents like $H_2S$, $CO_2$, $NH_3$ or any component having an optical absorption in the chosen wavelength region,

The emission spectrum of the light beams may be chosen so that the detector is able to detect two isotopes of a gaseous constituent. These isotopes may be carbon isotopes such as $^{12}C$ and $^{13}C$. They may be detected in gaseous constituent such as methane, ethane, propane, or other alkanes. The processor may calculate other parameters, such as the isotopic ratio of the gaseous constituents on the basis of the signal obtained from the detectors.

The analysis may comprise a flow controller for forming a gaseous flow so that the gaseous sample flows continuously in the photoacoustic cell(s). Emission of light beams and detection may be performed while the gaseous sample is flowing. Alternatively, the gaseous sample may be trapped in the cell while emission of light beams and detection are performed.

The quantification device may include a filtering device, comprising devices such as :

- a filter,
- an oven,
- a gas chromatograph comprising a separation column.

Filtering device may be situated upstream of the photoacoustic measurement unit, in order to selectively isolate one or several components prior to their compositional or isotopic analysis.

A method for analyzing a gaseous sample containing gaseous constituents extracted from a drilling fluid coming from a wellbore is also provided in the disclosure. The method comprises :

- extracting a gaseous sample from the drilling fluid,
- optionally, transporting the gaseous sample to the analytical device
- emitting a light beam having a predetermined emission spectrum in a photoacoustic cell where the gaseous sample is contained,
- detecting an acoustic signal due to the interactions of the light beam and the gaseous sample for the predetermined emission spectrum,
- calculating a parameter relative to a quantity of the gaseous constituents on the basis of a signal obtained from the detector.

The method may include emitting successively several light beams having distinct emission spectrum, and calculating the parameter relative to a quantity of a gaseous constituent on the basis of signals obtained from the detector and corresponding to the interaction of the gaseous sample with different light beams. Light beams may be emitted in the same and/or different cells.

The method may also include carrying a drilling fluid to a surface assembly situated outside of the wellbore.

Method may also include other features. It may be performed by the system according to the present disclosure.

## BRIEF DESCRIPTION OF THE DRAWINGS

Various aspects of this disclosure may be better understood upon reading the following detailed description and upon reference to the drawings in which:

FIG. 1 is a drawing of a schematic sectioned view of an analysis assembly according to some embodiments of the disclosure, arranged in an installation for drilling an oil well,

FIG.2 is a drawing of a schematic view of the analysis device of FIG. 1.

FIG.3 is a drawing of a schematic view of a photoacoustic measurement unit of the analysis device of FIG.2

FIG.4 is a drawing of a schematic view of a photoacoustic measurement device according to some embodiments of the disclosure

FIG.5A & FIG.5B are graphs representing the absorption spectra of different gaseous constituents in function of the wave number. Wave number on FIG.5A is between 2900 and 3020 and is between 1420 and 1520 on FIG.5B

FIG. 6 is a flow diagram of an analyzing method according to some embodiments of the disclosure.

**DETAILED DESCRIPTION**

One or more specific embodiments of the present disclosure will be described below. These described embodiments are examples of the presently disclosed techniques. Additionally, in an effort to provide a concise description of these embodiments, some features of an actual implementation may not be described in the specification. It should be appreciated that in the development of any such actual implementation, as in any engineering or design project, numerous implementation-specific decisions may be made to achieve the developers' specific goals, such as compliance with system-related and business-related constraints, which may vary from one implementation to another. Moreover, it should be appreciated that such a development effort might be complex and time consuming, but would still be a routine undertaking of design, fabrication, and manufacture for those of ordinary skill having the benefit of this disclosure.

When introducing elements of various embodiments of the present disclosure, the articles "a," "an," and "the" are intended to mean that there are one or more of the elements. The terms "comprising," "including," and "having" are intended to be inclusive and mean that there may be additional elements other than the listed elements. Additionally, it should be understood that references to "one embodiment" or "an embodiment" of the present disclosure are not intended to be interpreted as excluding the existence of additional embodiments that also incorporate the recited features.

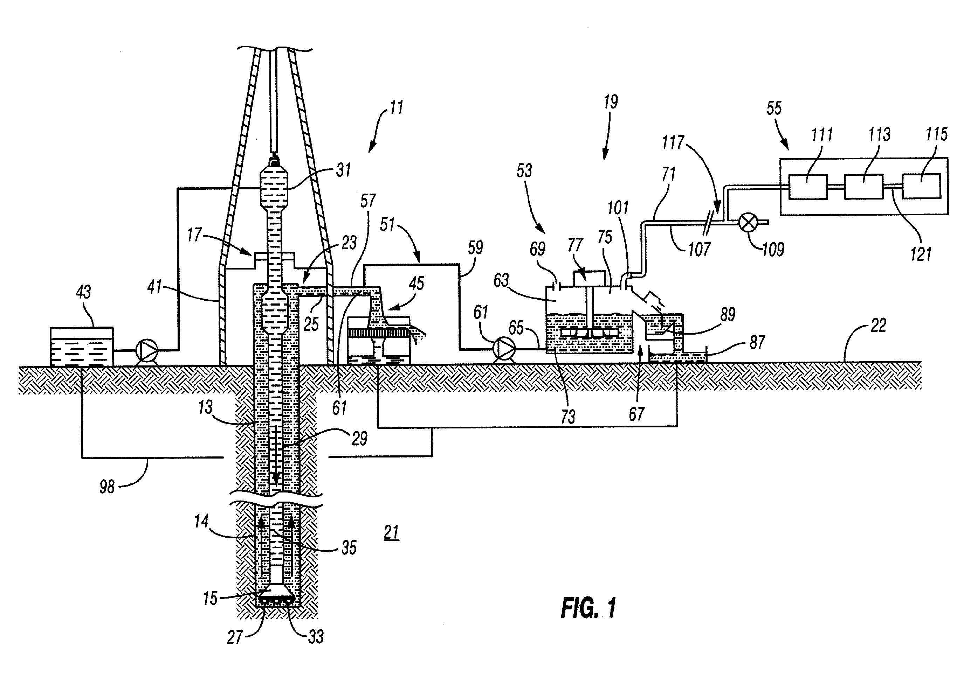

FIG.1 discloses an analysis assembly 9 used for the on-line analysis of the gaseous constituents of drilling muds in an installation 11 for drilling an oil production well.

As illustrated in FIG. 1, this installation 11 comprises a drilling pipe 13 in a borehole 14 through which a rotating drilling tool 15 extends, and a surface installation 17.

The drilling pipe comprises, in the region of the surface 22, a well head 23 which is provided with a pipe 25 for discharging a drilling fluid, referred to as drilling mud.

The drilling tool 15 comprises a drilling head 27, a drilling assembly 29 and a liquid injection head 31.

The drilling head 27 comprises a bit 33 for drilling through the rocks of the substratum 21. It is assembled in the lower portion of the drilling assembly 29 and is positioned at the bottom of the drilling pipe 13. The drilling head may also comprise an underreamer that enables to enlarge the hole formed by the bit (not shown on Figure 1).

The assembly 29 comprises an assembly of hollow drilling tubes also called drill string. These tubes delimit an internal space 35 which allows a fluid to be conveyed from the surface 22 to the drilling head 27. To this end, the liquid injection head 31 is screwed onto the upper portion of the assembly 29.

The surface installation 17 comprises means 41 for supporting and driving the drilling tool 15 in rotation, means 43 for injecting drilling fluid, also referred to as "mud" and a vibrating sieve 45.

The injection means 43 are hydraulically connected to the injection head 31 in order to introduce and circulate the drilling fluid in the inner space 35 of the drilling assembly 29. The drilling fluid is then directed to the discharge pipe 25 as shown on FIG. 1.

The vibrating sieve 45, also called shaker, collects the drilling fluid charged with drilling residues which is discharged from the discharge pipe 25 and separates the fluid from the solid drilling residues. Drilling fluid once separated from the solid drilling residues, is taken away from the well. Drilling fluid will afterwards be stored and/or recycled and/or treated.

The analysis assembly 9 comprises a sampling device 51 for sampling the mud, which means are tapped into the discharge pipe 25, a gas extraction device 53, and a device 55 for analyzing and quantifying the extracted gases.

The sampling device 51 comprise a liquid sampling head 57 which is tapped into the discharge pipe 25, a connection tube 59 and a peristaltic pump 61 whose flow rate can be adjusted.

The extraction device 53 comprises a vessel 63, a pipe 65 for conveying the mud into the vessel 63, a pipe 67 for discharging the mud from the vessel 63, an inlet 69 for introducing a carrier gas into the vessel 63, and a pipe 71 for extracting the extracted gases from the vessel 63.

The vessel 63 is formed by a sealed receptacle whose inner volume is, for example, between 0.4 and 3 liters. This vessel 63 comprises a lower portion 73 in which the mud circulates and an upper portion 75 which has a gaseous cap. The vessel 63 is further provided with an agitator 77 which is immersed in the mud.

The mud supply pipe 65 extends between the outlet of the peristaltic pump 61 and an inlet opening which is arranged in the lower portion 73 of the vessel 63.

This supply pipe 65 may be provided with a heater for heating the mud (not illustrated) in order to bring the temperature of this mud to values of between 25 and 120.degree. C., for instance between 60 and 90°C.

The discharge pipe 67 extends between an overflow passage which is arranged in the upper portion 75 of the vessel 63 and a retaining vessel 87 which is intended to receive the mud which is discharged from the device 53. It comprises a siphon in order to prevent gas from being introduced into the upper portion 75 of the vessel 63 via the discharge pipe 67. Gas is therefore introduced into the vessel 63 via the carrier gas introduction inlet 69. Carrier gas is an inert gas that does not include any of the constituent that should be detected by the quantification device, such as air or Helium for example.

The mud which is collected in the retaining vessel 87 is recycled towards the injection means 43 via a mud recirculation pipe 98.

The gas extraction pipe 71 extends between an extraction opening 101, which is arranged in the upper portion 75 of the vessel 63, and the analysis device 55. It comprises a transport line 107 which is provided with volume flow control means and suction means 109. The transport line 107 connects the vessel 63 which is arranged in the vicinity of the well head 23, in the explosive zone, to the analysis device 55 which is arranged with spacing from the well head 23 in a non-explosive zone, for example, in a pressurized cabin. It is normally at least 10 meters long. The transport line 107 may be made of polymer material such as PTFE or THV, as explained in patent No. US7232548 hereby incorporated by reference.

The suction means 109 comprise a vacuum pump which allows the gases extracted from the vessel 63 to be conveyed, by means of suction, to the analysis device 55.

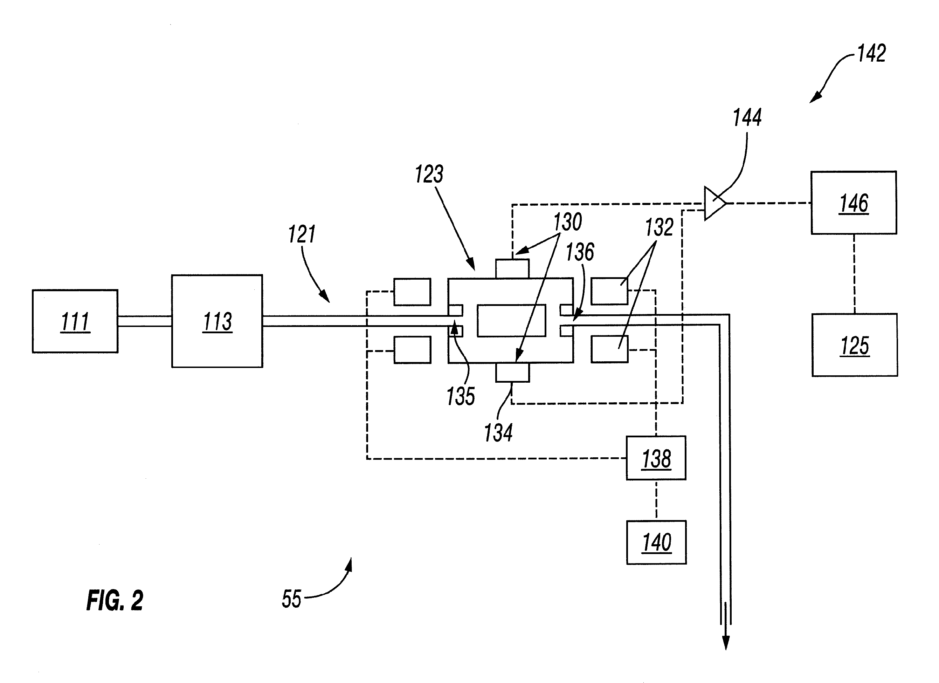

As illustrated in FIG. 2, the analysis device 55 according to the disclosure comprises an optional filtering device 111 for filtering undesirable gaseous constituents from the extracted gaseous sample, a flow controller 113 for forming a gaseous flow to be analyzed, and a quantification device 115 for quantifying the gaseous constituents to be analyzed in the drilling mud.

It also comprises a sampling pipe 117 which is tapped into the extraction pipe 71 in the vicinity of the pump 109, upstream of this pump.

The filtering device 111 may comprise a filter for retaining some constituents and preventing them to circulate towards the quantifying device 115, and/or a gas chromatograph comprising a separation column so that the different constituents of the gaseous sample are separated prior to their analysis in the quantifying device 115 (to avoid, for instance, analytical interferences between several components of the sample or to avoid to detect specific components) and/or a combustion and/or drying oven for eliminating some constituents of the gaseous sample. Such an oven may be at a temperature substantially between 900.degree. C. and 1100.degree. C.

The quantification device 115 includes a photoacoustic measurement unit 123 for performing photoacoustic detection and a control and calculation unit 125 which is connected electrically to the measurement unit 123. The calculation unit 125 may comprise a processor for executing the calculation operations. He may also include a memory associated with the processor for storing data coming from the photoacoustic measurement unit or other data in one or more database. The calculation unit 125 may be situated on-site or remote. It may include a computer, PDA and be connected to the photoacoustic measurement unit by a network.

Photoacoustic detection relies on periodically submitting molecules to a light beam, so that molecules absorb photons of the light beam, producing periodical increases of temperatures and pressure that create an acoustic wave. This acoustic wave is detected and may convey information relative to the molecules. The detected signal is for instance proportional to the amount of the molecules that have absorbed the photons in the cell.

The photoacoustic unit 123 comprises at least a photoacoustic cell 130, connected to the flow controller 113 and in which the gaseous sample extracted by the extraction device passes, at least a light source 132 for emitting a light beam into cell 130 so that the photons from the light beam are absorbed by the gaseous constituents situated inside the cell, and at least a detector 134 comprising a acoustoelectric transducer, such as a microphone, for detecting the acoustic wave produced following the absorption of photons by the gaseous sample.

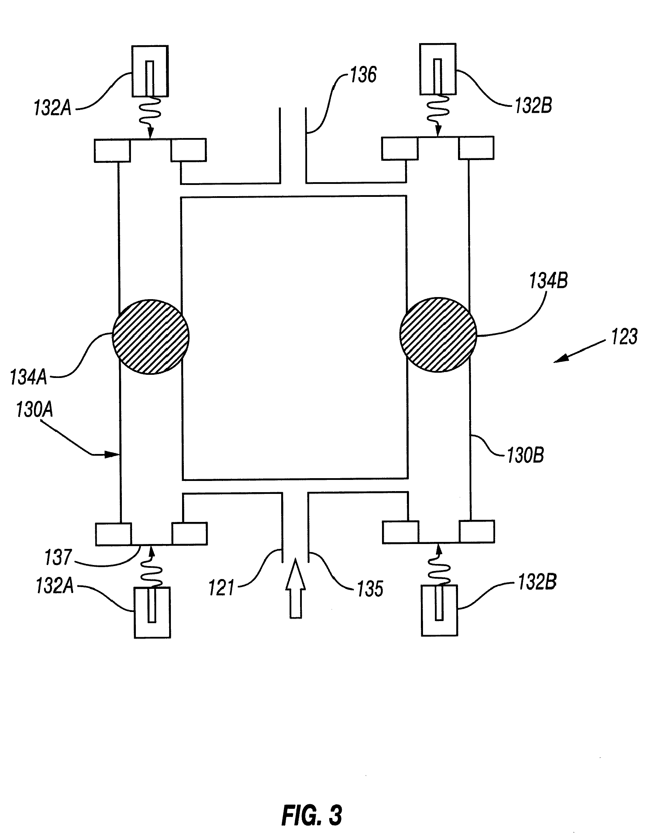

The photoacoustic unit illustrated in FIG.3 comprises two symmetric cells 130A, 130B of small volumes linked by two capillary tubes. The two cells 130 are Helmholtz resonators and are also connected to a supplying line 121, carrying the gas sample from flow controller to quantification device, by a gas inlet 135 at one side of the unit. A gas outlet 136 connecting both cells to the outside of the unit is also provided at the other side of the unit so that the gaseous sample flows into one of the cells 130 before exiting the unit 123. The cells are closed cavities except for the inlet 135 and outlet 136.

Two light sources 132A, respectively 132B are associated to each cell 130A, respectively 130B. Each source is provided at a longitudinal end of the cell 130. Cell 130 comprises walls having at least a transparent window 137 at each of its longitudinal end, on part or the totality of its surface, so that the light beam emitted by the light source reaches the inside of the cell. One or each of the light sources may comprise a LASER assembly, such as a semiconductor LASER assembly or a QCL (Quantum Cascade LASER) assembly so the emission spectrum of the beam is focused on one wavelength value and detection is accurate. A QCL LASER is for instance suited for detecting small amounts of a constituent as the continuous or pulsed power of a QCL assembly used as a light beam can be as high as several Watt. At least one of the light sources may be a tunable light source, having an adjustable emission spectrum. It may be a LASER such as a QCL LASER or but not limited to a black body or Globar source that may be combined with different filter for changing the emission spectrum. The light beams associated to the cells may have different emission spectrums so that the light beams may interact with different constituents. The spectrum of each of the light may comprise one or several wavelength values. The cell may also be associated with one light source, tunable or not, instead of a plurality.

As can be seen on Figure 2, the photoacoustic unit also comprises a modulator 138 associated to the light sources and a control unit 140 for powering and commanding the activation and modulation of the light sources. The modulator 138 may modulate the light source electrically (for instance by slightly varying the wavelength value at which the light beam is emitted) or mechanically (for instance by obscuring periodically the beam via a chopper). The modulating frequency may be a proper frequency (or resonant acoustic frequency) of each of the cell. The control unit 140 may command the light sources so that while a light source is turned on and emits a light beam, the other light sources are turned off and do not emit any light beams into the cell. The lights sources may be commanded so that they are successively turned on and off for instance. The control unit may comprise a timer.

One microphone 134 is also fitted on each cell body for detecting the acoustic waves in each of the cells. The signals obtained by each of the microphones 134A, 134B may be provided to a treatment unit 142 that collects and treats the signals obtained from the microphones to the calculation unit 125. Treatment unit 142 comprises a substractor 144. Indeed, signals obtained from the microphone associated with the cell in which no light source is turned on are substracted from the signals obtained from the microphone associated with the cell in which a light source is turned on so that an acoustic wave is produced into the cells due to the interactions of the gaseous sample and the photons of the light beam. Thus the signal corresponding to outside noises will be zeroed and the signal coming from the gas will be measured without taking these noises into account. Further, if the modulation of the laser is done at a frequency corresponding to the resonant acoustic frequency of the cell, the signal on microphones 134A and 134B generated by the absorption of light by the gas are in phase opposition. The subtracting of microphones' signals thus further results in an increase of the gas signal that gives an enhanced sensitivity. Treatment unit 142 may also comprise an amplifier, a filter 146. for eliminating the noise of the signal, etc.

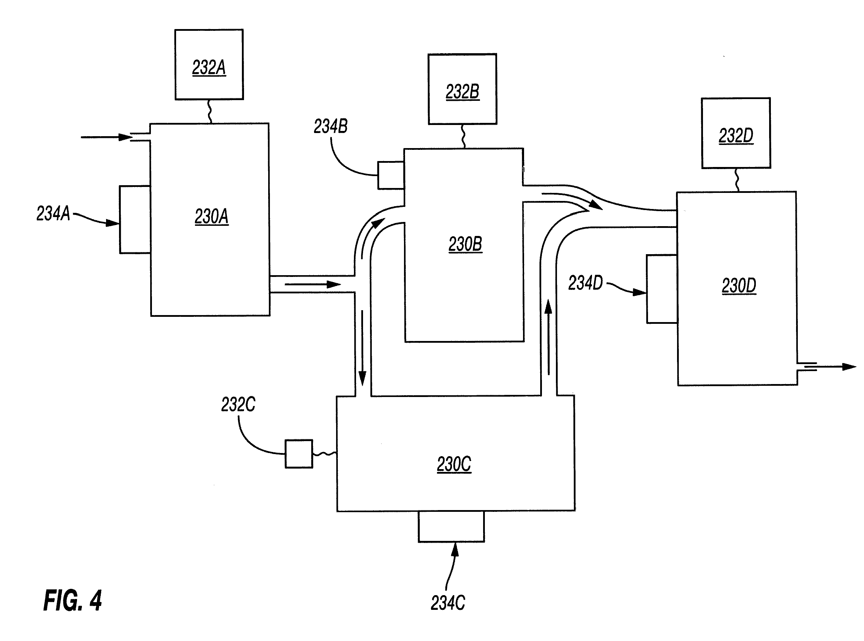

The disclosed cell is an example among others and is described in more details in document No. WO2003/83455 hereby incorporated by reference. Other photoacoustic measurement units may however be used such as a measurement unit comprising one cavity with a gas inlet and gas outlet, a light source, tunable or not, and a detector. Any unit can be combined in series or parallel with other units featuring different light sources as illustrated on FIG.4 for instance where cell 230A is in series with cells 230B & 230C, arranged in parallel, and then with cell 230D. Light sources 232A-232D may be of different types and/or producing light beams having different emission spectrums. When cells are provided in series the control unit may command the activation of the light sources on the basis of data obtained by measurement devices on the gas flow velocity so that the signals acquired by detectors associated to each cell relate substantially to the same gaseous sample.

The emission spectrums of at least some of the light beams are chosen so that the emitted photons are absorbed by molecules comprising at least a carbon atom, such as alkanes comprising less than fifteen carbon atoms. They may be chosen to be absorbed by methane and/or methane (C1), ethane (C2), propane (C3) such as n-propane (nC3) or isopropane (iC3), butane (C4) such as isobutane (iC4) or other isomers of butane (nC4), pentane (C5) such as isopentane (iC5) or other isomers of pentane (nC5). They may also be chosen to enable detection of two isotopes of a gaseous constituent such as two isotopes of a carbon atom ($^{12}$C or $^{13}$C) in alkanes such as methane, ethane or propane for instance.

The emission spectrums of the light sources may be chosen in the infra-red portion of the electromagnetic spectrum. Some emission spectrums may be chosen so that one gaseous constituent absorbs the photons emitted at this spectrum, in particular when the spectrum is focused on one wavelength. Other wavelength values may be chosen so that several gaseous constituents absorb the photons emitted at this spectrum.

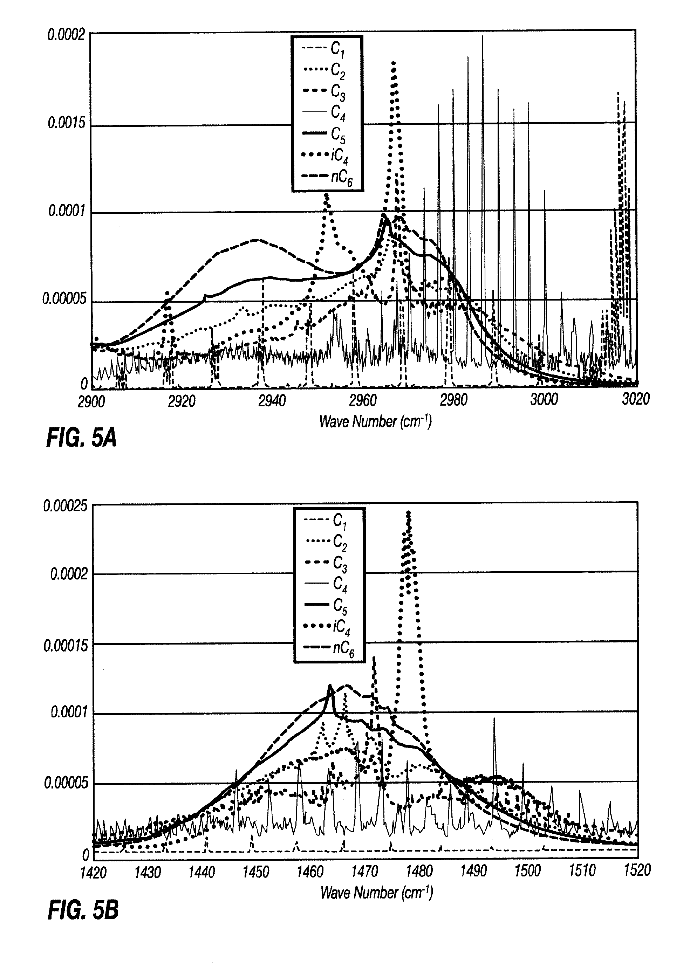

Wavelength values that may be used in the quantification device are given to the table below. Corresponding wave number (wavelength's invert) is also featured in the table. The wavelength values situated in the area around 3.37 $\mu$m (2960 cm$^{-1}$) and around 6.80 $\mu$m (1470 cm$^{-1}$) are absorbed by most of the alkanes, as may be seen on Figures 5A and 5B and may therefore be a good choice for detecting these alkanes. For some compounds, in particular the ones containing $^{13}$C, other wavelength values, situated in the area between 7.52 and 7.70 $\mu$m (1300 to 1330 cm$^{-1}$), between 1, 58 and 1, 67 $\mu$m (6000 to 6300 cm$^{-1}$), between 12,05 and 12,34 $\mu$m (810 to 830 cm$^{-1}$) or between 6.67 and 6.75 $\mu$m (1480 a 1500 cm$^{-1}$). So other wavelengths that the ones given in the table below may be chosen in these areas or in other areas.

| Wavelength ($\mu$m) | 1.65 | 6.70 | ~3.369 | ~3.387 | ~3.372 | ~3.401 |
|---|---|---|---|---|---|---|
| Wavenumber (cm$^{-1}$) | ~6061 | ~1493 | 2968 | 2952 | 2966 | 2940 |
| Absorbing gaseous constituents | $^{12}$C1 | $^{12}$C2 | C3, iC4 | iC4 | C3, iC4,n C4 | C5 |

In an embodiment for the photoacoustic measurement unit 123, the light sources 132 are as follows :

- 　A LASER emitting a light beam at a wavelength between 1, 58 and 1, 67 $\mu$m (6000 to 6300 cm$^{-1}$) for detecting $^{12}$C1,
- 　A LASER emitting a light beam at a wavelength between 7.52 and 7.70 $\mu$m (1300 to 1330 cm$^{-1}$) for detecting $^{13}$C1,
- 　A LASER emitting a light beam at a wavelength between 6.67 and 6.75 $\mu$m (1480 a 1500 cm$^{-1}$) for detecting $^{12}$C2,
- 　A LASER emitting a light beam at a wavelength between 12,05 and 12,34 $\mu$m (810 to 830 cm$^{-1}$)for detecting $^{13}$C2.

Such a photoacoustic measurement unit would enable to obtain the concentration of the carbon isotopes in methane and ethane. An isotopic ratio may then be calculated by the calculation unit 125. A QCL LASER may be used for each of these wavelengths and in particular for the wavelengths that are absorbed by $^{13}$C isotopes generally present in lesser amount in the sample.

In another embodiment for the photoacoustic measurement unit 123, the light sources 132 are as follows :

- A LASER emitting a light beam at a wavelength of 1.65 $\mu$m (for detecting $^{12}$C1),
- A LASER emitting a light beam at a wavelength of 6.70 $\mu$m (for detecting $^{12}$C2),
- A tunable LASER centered around 3.37 $\mu$m and emitting light beams with wavelengths between 3.35 to 3.41 $\mu$m, to scan particular wavelengths of interest (for detecting C3, iC4, nC4, C5). These wavelength values may be the ones listed in the table above.

Such a photoacoustic measurement unit would enable to obtain the concentrations of the C1-C5 alkanes in the gaseous sample.

As in this embodiment, at several wavelengths, more than one gaseous sample is detected, the calculation unit 125 comprises a post-processing unit in order to determine the concentration of each gaseous constituent in function of the signals received from the detector at each wavelength. The post-processing unit then solves an equation system such as the following:

$$\forall\ w_j, \sum_{i=1}^{n} A_{ij} \times x_i = X_j$$

$w_j$ is a predetermined wavelength value, $X_j$ the total amount (here concentration) of gas of interest detected for wavelength value $w_j$, $x_i$ the amount of a gaseous constituent i and $A_{ij}$ is the fraction of the signal due to the constituent i at wavelength $w_j$. Coefficients $A_{ij}$ may have been determined beforehand by a calibration including measuring predetermined mix of gaseous constituents. The embodiment that was described above takes into account C1-C5 measurements but of course the concentration of other heavier alkanes may be determined via the same technique, using a greater number of measured wavelength values. These wavelength values may be obtained in the above-mentioned areas around 3.37 and 6.80 $\mu$m.

Any combination of light sources described in the two embodiments may of course be implemented in one or several cells. The light beams may also be emitted at other wavelength values that enable detection of one or several alkanes. Other gaseous constituents may also be detected and quantified according to the same technique provided the wavelengths at which the photons from the light beams are emitted are chosen so that the photons are absorbed by the constituent.

A light source emitting a light beam having a emission spectrum comprising several wavelengths may also be used, in which case the equation system as follows is solved:

$$\sum_{j=1}^{m} B_j \sum_{i=1}^{n} A_{ij} \times x_i = \sum_{j=1}^{m} X_j$$

$X_j$ is the total concentration of gas of interest detected for wavelength value $w_j$ (light beam comprising wavelengths $w_1, ..., w_m$), $x_i$ the concentration of a gaseous constituent i, $A_{ij}$ is the fraction of the signal due to the constituent i at wavelength $w_j$ and $B_j$ is the fraction of photons of the emission spectrum at wavelength $w_j$. $A_{ij}$ may be determined beforehand via a calibration and $B_j$ is known from the emission spectrum of the light beam.

Several light sources may also emit light beams at the same time in the cell so that the two light beams form a resulting light beam having a resulting spectrum. Post-processing unit of calculation unit 125 may determine resulting spectrum and solve the above-mentioned system of equation taking into account the resulting light beam, in particular for coefficients $B_j$.

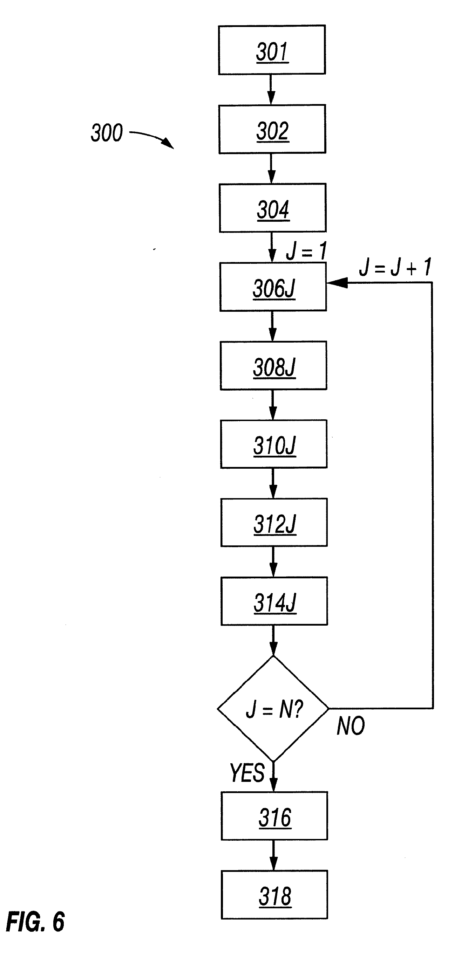

A method 300 according to an embodiment of the disclosure for quantifying a constituent which is contained in a gaseous sample taken from a drilling mud and which is carried out on-line when a well is drilled will now be described with reference to FIG.6.

In order to carry out the drilling operation, the drilling tool 15 is driven in rotation by the surface installation 41.

A drilling liquid is introduced into the inner space 35 of the drilling assembly 29 by the injection means 43. This liquid moves downwards as far as the drilling head 27 and passes into the drilling pipe 13 through the drilling head 27. This liquid cools and lubricates the drilling means 33. Then the liquid collects the solid debris resulting from the drilling operation and moves upwards again through the annular space which is defined between the drilling assembly 29 and the walls of the drilling pipe 13, then is discharged via the discharge pipe 25.

The peristaltic pump 61 is then activated in order to remove, in a continuous manner, a specific fraction of the drilling mud which is circulating in the pipe 25.

This fraction of mud is conveyed as far as the chamber 63 via the supply pipe 65.

The agitator 77 is driven in rotation in the lower portion 73 of the chamber 63 in order to bring about the extraction of the gases contained in the mud and the mixture of the extracted gases with the carrier gas drawn through the injection inlet 69.

The gaseous mixture is extracted at 301 via the extraction pipe 71, under the action of the suction produced by the vacuum pump 109. This mixture is then conveyed as far as the analysis device 55.

When the gas sample arrives in the analysis device, it may first be filtered or dried at 302 by filtering device 111, for instance to remove water from the gas sample. Other constituents that may be considered as perturbation for the photoacoustic measurement unit 123 may be also filtered as explained above. The sample is then directed to the mass flow controller 113 so that a gaseous flow is formed at 304. The mass flow controller allowing a stable and steady gas flow. The gas sample then enters into the photoacoustic cell.

Once the gas sample is in the cell, a first light source 132A focused at a first wavelength value is turned on and modulated at the acoustic resonant frequency of the cell for a period of 1-10 seconds in order to get a stable signal, at $306_1$. The detector 134 then detects an acoustic wave generated in the cell, at $308_1$. The signal obtained from the detector 134A, 134B is then forwarded to the treatment unit 142 and to the calculation unit so that a concentration of gaseous constituents is obtained from the signal of the detectors 134A, 134B for said first wavelength value, at $310_1$. This concentration of gaseous constituents for the first wavelength value is stored at $312_1$ in the calculation unit along with data relative to the first wavelength that are also forwarded to the calculation unit 125. First light source is then turned off or light beam at first wavelength value is interrupted at $314_1$.

The light sources associated with the photoacoustic cells are turned on successively, i.e. same sequence $306_j$-$314_j$ is repeated for light beams at second, third, fourth, etc. wavelength values by turning on again the first light source 132A and/or the other light sources 132B. In fact, for each wavelength values $w_j$ (j=1...N), sequence $306_j$-$314_j$ is repeated. The flow controller controls the flow so that the gaseous sample that is situated in the cell when the first light beam focused on the first wavelength value is substantially the same as the one situated in the cell when the Nth and last light beam of the sequence focused on the Nth wavelength value is emitted. This is normally the case in drilling application as the gaseous constituents of the gas sample are changing continuously.

Alternatively, the cell 130 may be filled out with the gaseous sample and then isolated, for instance via valves that are added in the inlet and in the outlet of the cell, measurements being performed while the cell is isolated. Cell is opened again (for instance, at the outlet first and at the inlet once the gaseous sample has been evacuated) once the measurements have been performed.

If several other light sources are associated with other cells that are in series with a first cell, a timer is launched so that the control unit turns on the light sources associated with this cell when the gas sample that was in the first cell when the associated light sources were turned on is substantially the same as the gas sample that is in the second cell when the associated light sources are turned on. The time set by the timer may depend from the flow of the gas sample obtained from the mass flow controller or other flow sensors.

Calculation unit 125 then determines at 316 the concentration of each of the gaseous constituent as explained above via the post-processing unit and a database storing the coefficients Aij for each wavelength, the coefficients having been obtained by a calibration performed beforehand via one or several predetermined gas sample comprising a known mix of different gaseous constituents.

From these calculated concentrations, other parameters, such as isotopic ratio of a gaseous constituent may be obtained at 318. The analysis device 55 provides a full set of measurements in 4-40 seconds.

In view of the entirety of the present disclosure, including the figures, a person skilled in the art should appreciate that they may readily use the present disclosure as a basis for designing or modifying other processes and structures for carrying out the same uses and/or achieving the same aspects introduced herein. A person skilled in the art should also realize that such equivalent constructions do not depart from the spirit and scope of the present disclosure, and that they may make various changes, substitutions and alterations herein without departing from the spirit and scope of the present disclosure. For example, although the preceding description has been described herein with reference to particular means, materials and embodiments, it is not intended to be limited to the particulars disclosed herein; rather, it extends to functionally equivalent structures, methods, and uses, such as are within the scope of the appended claims.

For instance the quantifying device may have a different architecture from what has been disclosed. The shape of the cell may be circular, parallelepiped, etc. and the light source may be positioned so that the light beams passes through a lateral wall of the cell for instance. When cells are in parallel or in series, the cells may not be of the same shape and dimensions. The light sources may differ from what has been disclosed, as the wavelength values. The method may also differ from what has been disclosed as some disclosed elements of the methods are optional. The order of the method is not limited to what has been disclosed.

**Claims**

1. A system for analyzing a gaseous sample extracted from a drilling fluid coming from a wellbore, wherein the system comprises a surface assembly, situated outside of the wellbore and including :

   - an extractor for extracting a gaseous sample from the drilling fluid,
   - a quantification device connected to the extractor for quantifying at least a gaseous constituent contained the gaseous sample,
   wherein the quantification device comprises at least a photoacoustic cell in fluid communication with the extractor, at least a light source for emitting a light beam at a predetermined emission spectrum in the photoacoustic cell, at least an acoustic detector positioned in the photoacoustic cell and a processor for calculating a parameter relative to the quantity of the gaseous constituent on the basis of a signal obtained from the detector.

2. System according to claim 1, wherein said light sources are configured to emit at least two light beams having a distinct emission spectrum.

3. System according to claim 2, wherein it comprises a plurality of light sources , each of the light sources emitting a light beam, so that at least two of the light beams have a distinct emission spectrum.

4. System according to any one of claim 2 or 3, wherein at least one of the light sources having an adjustable emission spectrum.

5. System according to any preceding claims, wherein one or more of the light sources include a LASER assembly.

6. System according to claim 5, wherein one or more of the LASER assembly is a QCL (Quantum Cascade LASER) LASER assembly.

7. System according to any of the preceding claim, wherein the power of at least one of the light source is greater than 1 mW

8. System according to any one of the preceding claims, wherein the processor is configured to calculate the parameter relative to the quantity of a gaseous constituent in view of a first signal obtained from the acoustic detector corresponding to the emission of a first light beam and a second signal obtained from the acoustic detector corresponding to the emission of a second light beam.

9. System according to the preceding claim, wherein the processor is configured to calculate the parameter relative to the quantity of at least a gaseous constituent in view of the following formula :

$$\forall \, w_j, \sum_{i=1}^{n} A_{ij} \times x_i = X_j$$

Wherein $w_j$ a predetermined wavelength value, $X_j$ the total amount of gas detected for wavelength value $w_j$, $x_i$ the amount of a gaseous constituent i and $A_{ij}$ is the fraction of the signal due to the constituent i at wavelength $w_j$.

10. System according to any one of the preceding claims comprising at least two photoacoustic cells in parallel or in series.

11. System according to any one of the preceding claims wherein at least one of the wavelength values of the light sources are chosen in the following areas :

- Between 3.30 and 3.45 $\mu$m,
- Between 6.67 and 6.75 $\mu$m
- Between 1, 58 and 1, 67 $\mu$m
- Between 7.52 and 7.70 $\mu$m
- Between 12, 05 and 12,34 $\mu$m.

12. System according to any one of the preceding claims wherein the emission spectrum of the light beams are chosen so that the detector is able to detect gaseous constituents comprising at least one atom of carbon, including in particular an alkane with less than 15 carbon atoms and in particular methane (C1), ethane (C2), propane (C3) such as n-propane or isopropane, butane (C4) such as isobutane or other isomers of butane, pentane (C5) such as isopentane or other isomers of pentane.

13. System according to any one of the preceding claims wherein the emission spectrum of the light beams are chosen so that the detector is able to detect two isotopes of at least a gaseous constituent such as comprising at least one atom of carbon and in particular methane , ethane or propane, the processor being configured to calculate the isotopic ratio of the gaseous constituents on the basis of the signal obtained from the detectors.

14. A method for analyzing a gaseous sample containing at least a gaseous constituent extracted from a drilling fluid coming from a wellbore, comprising :

- extracting a gaseous sample from the drilling fluid with a surface assembly situated outside of the wellbore,
- emitting at least a light beam having a predetermined emission spectrum in a photoacoustic cell where the gaseous sample is contained,
- detecting an acoustic signal due to the interactions of the light beam and the gaseous sample for the predetermined emission spectrum,
- calculating a parameter relative to the quantity of at least one of the the gaseous constituent on the basis of a signal obtained from the detector.

15. The method of claim 14,comprising emitting successively several light beams having distinct emission spectrum, and calculating the parameter relative to the quantity of at least a gaseous constituent on the basis of signals obtained from the detector and corresponding to the interaction of the gaseous sample with at least two of the light beams.

FIG. 1

EP 3 012 616 A1

EP 3 012 616 A1

FIG. 2

**FIG. 3**

*FIG. 4*

FIG. 5A

FIG. 5B

FIG. 6

Europäisches
Patentamt

European
Patent Office

Office européen
des brevets

# EUROPEAN SEARCH REPORT

Application Number

EP 14 29 0322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/266108 A1 (DIFOGGIO ROCCO [US]) 30 November 2006 (2006-11-30) | 1,4,5,7, 11-14 | INV. G01N21/17 |
| Y | * paragraphs [0002], [0018], [0020], [0021], [0026], [0030], [0031], [0034] * <br> * figures 1,6 * | 6 | G01N33/28 E21B49/00 |
| X | US 4 492 862 A (GRYNBERG JACK [US] ET AL) 8 January 1985 (1985-01-08) <br><br> * column 1, line 29 - line 33 * <br> * column 6, line 14 - column 7, line 48 * <br> * column 9, line 43 - line 68 * <br> * column 15, line 7 - line 18 * <br> * figure 1 * | 1-3,5, 7-10,14, 15 | |
| Y | GB 2 492 841 A (SECR DEFENCE [GB]) 16 January 2013 (2013-01-16) | 6 | |
| A | * page 4, line 7 - line 14 * <br> * page 7, line 1 - line 6 * <br> * page 12, line 6 - line 20 * <br> * page 17, line 6 - line 8 * | 11 | |

TECHNICAL FIELDS
SEARCHED (IPC)

G01N
E21B

The present search report has been drawn up for all claims

| Place of search | Date of completion of the search | Examiner |
|---|---|---|
| Munich | 23 March 2015 | D'Alessandro, Davide |

EPO FORM 1503 03.82 (P04C01)

## ANNEX TO THE EUROPEAN SEARCH REPORT
## ON EUROPEAN PATENT APPLICATION NO.

EP 14 29 0322

This annex lists the patent family members relating to the patent documents cited in the above-mentioned European search report.
The members are as contained in the European Patent Office EDP file on
The European Patent Office is in no way liable for these particulars which are merely given for the purpose of information.

23-03-2015

| Patent document cited in search report | | | Publication date | Patent family member(s) | | | Publication date |
|---|---|---|---|---|---|---|---|
| US | 2006266108 | A1 | 30-11-2006 | CN | 101203658 | A | 18-06-2008 |
| | | | | US | 2006266108 | A1 | 30-11-2006 |
| US | 4492862 | A | 08-01-1985 | EP | 0072222 | A1 | 16-02-1983 |
| | | | | US | 4492862 | A | 08-01-1985 |
| GB | 2492841 | A | 16-01-2013 | GB | 2492841 | A | 16-01-2013 |
| | | | | WO | 2013011253 | A1 | 24-01-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7232548 B
- WO 200383455 A